# EUROPÄISCHE PATENTSCHRIFT

(11) **EP 0 594 027 B1**
(45) Veröffentlichungstag und Bekanntmachung des Hinweises auf die Patenterteilung: **21.08.1996**
(21) Anmeldenummer: 93116435.4
(22) Anmeldetag: 11.10.1993
(51) Int. Cl.: C07C 315/04, C07C 317/44

(54) **Verfahren zur Herstellung von Methylsulfonylbenzoesäuren**
Method for the preparation of methylsulphonylbenzoic acids
Procédé pour la préparation des acides méthylsulfonylbenzoiques

(30) Priorität: 19.10.1992 DE 4235155
(43) Veröffentlichungstag der Anmeldung: 27.04.1994
(73) Patentinhaber: BASF Aktiengesellschaft, 67063 Ludwigshafen (DE)
(72) Erfinder: Hagen, Helmut, Dr., D-6710 Frankenthal (DE)

(56) Entgegenhaltungen:
- EP-A- 0 460 544
- WO-A-90/06302

## Beschreibung

Die vorliegende Erfindung betrifft ein neues Verfahren zur Herstellung von Methylsulfonylbenzoesäuren durch Oxidation der entsprechenden Methylsulfonyltoluole.

Aus der EP-A-460 544 ist die Herstellung von Hydroxylsulfonylanthranilsäuren durch Oxidation der entsprechenden Nitrotoluole bekannt. Ferner wird in der WO-A 90/06302 die Herstellung von Halogenylsulfonylbenzoesäuren aus den entsprechenden Toluolen durch Oxidation mit Salpetersäure beschrieben.

Aufgabe der vorliegenden Erfindung war es, ein neues Verfahren zur Herstellung von Methylsulfonylbenzoesäuren bereitzustellen. Das neue Verfahren sollte mit Methylsulfonyltoluolen als Edukten durchgeführt werden, wobei durch Oxidation der toluolischen Methylgruppe die Zielprodukte in guter Ausbeute und hoher Reinheit erhalten werden sollten.

Es wurde nun gefunden, daß die Herstellung von Methylsulfonylbenzoesäuren der Formel I 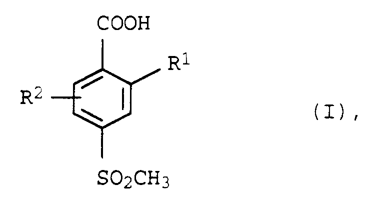 in der
- R¹: Wasserstoff, Nitro, Halogen oder Hydroxysulfonyl und
- R²: Wasserstoff, Nitro oder Halogen bedeuten, vorteilhaft gelingt, wenn man Methylsulfonyltoluole der Formel II 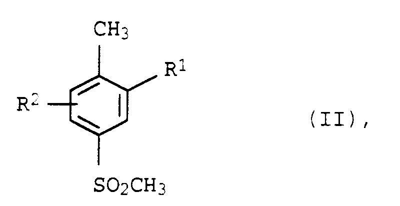
in der R¹ und R² jeweils die obengenannte Bedeutung besitzen, in Schwefelsäure in Gegenwart von Vanadium- oder Kobaltverbindungen mit Salpetersäure und Luft oxidiert.

Reste R¹ und R² sind z.B. Fluor, Chlor oder Brom.

Geeignete Vanadiumverbindungen sind insbesondere Vanadium(V)-Verbindungen, wie Vanadiumpentoxid oder Ammoniummetavanadat (NH₄VO₃). Die Verwendung von Vanadiumpentoxid ist bevorzugt. Unter den Reaktionsbedingungen liegen die Vanadiumverbindungen in der Regel als Vanadium(V)sulfat vor.

Geeignete Kobaltverbindungen sind insbesondere Kobalt(II)verbindungen, wie Kobalt(II)sulfat-heptahydrat, Kobalt(II)nitrat-hexahydrat oder Kobalt(II)acetat-tetrahydrat.

Die Durchfuhrung des erfindungsgemäßen Verfahrens in Gegenwart von Vanadiumverbindungen ist bevorzugt.

Das erfindungsgemäße Verfahren wird in Schwefelsäure als Reaktionsmedium durchgeführt. Vorteilhaft verwendet man dabei 60 bis 80 gew.-%ige, insbesondere 70 bis 75 gew.-%ige Schwefelsäure. Bezogen auf das Gewicht an Methylsulfonyltoluol II verwendet man in der Regel 100 bis 1200 Gew.-%, vorzugsweise 500 bis 1000 Gew.-% Schwefelsäure.

Als Oxidationsmittel dienen Salpetersäure und Luft. Vorteilhaft verwendet man dabei 30 bis 100 gew.-%ige, insbesondere 55 bis 65 gew.-%ige, Salpetersäure.

Bezogen auf das Gewicht an Methylsulfonyltoluol II verwendet man in der Regel 100 bis 400 Gew.-%, vorzugsweise 200 bis 300 Gew.-%, Salpetersäure. Diese Menge an Salpetersäure wird dabei, über den Reaktionsverlauf (in der Regel 6 bis 12 Stunden) verteilt, gleichmäßig zum Reaktionsgemisch dosiert.

Die neben Salpetersäure als weiteres Oxidationsmittel verwendete Luft wird in der Regel in einer Menge von 10 bis 100 l/h, vorzugsweise 20 bis 50 l/h, jeweils bezogen auf 1 l Reaktorvolumen, angewandt. Diese Menge wird dabei, über den Reaktionsverlauf verteilt, gleichmäßig in das Reaktionsgemisch geleitet.

Die Vanadium- oder Kobaltverbindungen kommen im allgemeinen in katalytischer Menge zur Anwendung. Bezogen auf das Gewicht von Methylsulfonyltoluol der Formel II wendet man dabei üblicherweise 1 bis 10 Gew.-%, vorzugsweise 2 bis 5 Gew.-%, Vanadium- oder Kobaltverbindung an.

Das neue Verfahren wird bei einer Temperatur von 130 bis 170°C, vorzugsweise 145 bis 155°C, vorgenommen, wobei in der Regel atmosphärischer Druck herrscht.

Bevorzugt wird das erfindungsgemäße Verfahren zur Herstellung von Methylsulfonylbenzoesäuren der Formel I angewandt, in der R¹ Wasserstoff oder Nitro und R² Wasserstoff bedeutet.

Das neue Verfahren wird zweckmäßig so vorgenommen, daß man Methylsulfonyltoluol II, Vanadium- oder Kobaltverbindung und Schwefelsäure vorlegt und unter Einleiten von Luft auf die obengenannte Temperatur erhitzt. Dann wird mit der Zudosierung der Salpetersäure begonnen. Vorteilhaft wird gleichzeitig verdünnte (ca. 20 bis 40 gew.-%ige) Salpetersäure aus dem Reaktionsgemisch abdestilliert.

Nach beendeter Umsetzung wird das Reaktionsgemisch abgekühlt und die als Niederschlag anfallende Methylsulfonylbenzoesäure abgesaugt, mit Wasser gewaschen und getrocknet.

Die als Edukte dienenden Methylsulfonyltoluole der Formel II können auf an sich bekannte Weise hergestellte werden. Beispielsweise kann man die entsprechenden Toluole in einer Friedel-Crafts-Reaktion mit Methansulfonsäurechlorid umsetzen.

Besonders vorteilhaft ist jedoch die Kombination des erfindungsgemäßen Verfahrens mit der aus der WO 90/06301 bekannten Herstellmethode für Methylsulfonyltoluol.

Dabei geht man von Toluolsulfonsäurechloriden der Formel III 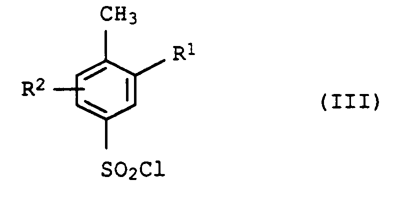 aus, in der R¹ und R² jeweils die obengenannte Bedeutung besitzen, und reduziert sie in wäßriger Lösung mit einem Alkalisulfit, insbesondere Natriumsulfit. Das resultierende Alkalisulfinat wird dann mit Chloressigsäure alkyliert und durch Erwärmen decarboxyliert.

Die Reduktion des Toluolsulfonsäurechlorids III erfolgt in wäßriger Lösung mit Natriumsulfit in Gegenwart von Natriumhydrogencarbonat bei einer Temperatur von 15 bis 30°C.

Nach Zugabe einer wäßrigen Lösung des Natriumsalzes der Chloressigsäure wird das Reaktionsgemisch 15 bis 20 Stunden unter Rückfluß gerührt. Nach dem Abkühlen fällt das Methylsulfonyltoluol II als Niederschlag an, der abgesaugt und ohne weitere Reinigung als Edukt im erfindungsgemäßen Verfahren verwendet werden kann.

Die für die Herstellung derjenigen Methylsulfonylbenzoesäuren der Formel I, in der R¹ Nitro bedeutet, als Ausgangsprodukte dienende Methylsulfonyltoluole II, gewinnt man vorteilhaft durch Nitrierung der entsprechenden nitrogruppenfreien Methylsulfonyltoluole II. Dabei wird Methylsulfonyltoluol II mit 50 bis 65 gew.-%iger Salpetersäure bei einer Temperatur von 0 bis 10°C in konz. Schwefelsäure nitriert. Nach beendeter Nitrierung wird das Reaktionsgemisch mit Wasser verdünnt bis die Schwefelsäure als 60 bis 80 gew.-%ige, vorzugsweise als ca. 70 gew.-%ige wäßrige Schwefelsäure vorliegt. Dieses Gemisch kann dann direkt für die Durchführung des erfindungsgemäßen Verfahrens verwendet werden.

Das erfindungsgemäße Verfahren, das sowohl in kontinuierlicher als auch diskontinuierlicher Arbeitsweise vorgenommen werden kann, liefert die Methylsulfonylbenzoesäuren der Formel I in einfacher Weise und in guter Ausbeute und hoher Reinheit.

Überraschend ist, daß unter den erfindungsgemäßen Bedingungen die kernständige Methylgruppe weitgehend selektiv zur Carboxylgruppe oxidiert wird, während die Methylsulfonylgruppe praktisch nicht angegriffen wird.

Die mittels des neuen Verfahrens erhältlichen Methylsulfonylbenzoesäuren sind wertvolle Zwischenprodukte für die Herstellung von Farbstoffen oder Wirkstoffen, insbesondere für die Herstellung von Herbiziden, z.B. wie in der US-A- 5 055 605 beschrieben.

Die folgenden Beispiele sollen die Erfindung näher erläutern.

### Beispiel 1

a) Herstellung von 4-Methylsulfonyltoluol
   176,4 g Natriumsulfit und 226 g Natriumhydrogencarbonat wurden in 1800 ml Wasser gelost und auf 15°C abgekühlt. Bei dieser Temperatur gab man unter gutem Rühren 244 g p-Toluolsulfonsäurechlorid zu. Das Gemisch wurde noch 3 Stunden bei 15°C und über Nacht bei Raumtemperatur gerührt. Anschließend wurde auf 40°C erwärmt und eine Losung von 175 g Natriumsalz der Chloressigsäure in 400 ml Wasser zugegeben. Das Reaktionsgemisch wurde für 24 Stunden unter Rückfluß gerührt. Nach Abkühlen auf 5 bis 10°C wurde das ausgefallene Produkt abgesaugt und bei 50°C unter vermindertem Druck getrocknet. Man erhielt 192,5 g 4-Methylsulfonyltoluol ( Fp.: 83 bis 86°C).
b) Herstellung von 4-Methylsulfonyl-2-nitrotoluol
   68 g 4-Methylsulfonyltoluol wurden in 400 ml konz. Schwefelsäure bei 0 bis 10°C mit einer Mischung aus 80 ml konz. Schwefelsäure und 31 ml 65 gew.-%iger Salpetersäure versetzt. Man rührte das Reaktionsgemisch 2 Stunden bei 10°C und 2 Stunden bei Raumtemperatur. Anschließend wurde auf ca. 1 kg Eis gegossen, der Feststoff abgesaugt, mit Wasser nachgewaschen und unter vermindertem Druck bei 50°C getrocknet. Man erhielt 85 g 4-Methylsulfonyl-2-nitrotoluol (Fp.: 112 bis 115°C).
c) Herstellung von 4-Methylsulfonyl-2-nitrobenzoesäure
   45 g 4-Methylsulfonyl-2-nitrotoluol und 1 g Vanadiumpentoxid wurden in 375 ml 70 gew.-%iger Schwefelsäure gelöst. Das Reaktionsgemisch wurde unter Einleiten von Luft (100 l/h) auf 145°C erhitzt. Bei dieser Temperatur wurden 0,25 ml/min 65 gew.-%ige Salpetersäure zudosiert. Nach 9 Stunden waren insgesamt 135 ml Salptersäure zugegeben. Gleichzeitig wurden in diesem Zeitraum aus dem Reaktionsgemisch 120 ml 35 gew.-%ige Salpetersäure abdestilliert.
   Das Reaktionsgemisch wurde dann auf 20°C abgekühlt, der Niederschlag abgesaugt und mit Wasser gewaschen.
   Man erhielt 44 g (83 % d. Theorie) 4-Methylsulfonyl-2-nitrobenzoesäure (Fp.: 204 bis 206°C).

### Beispiel 2

68 g 4-Methylsulfonyltoluol (Beispiel 1a) wurden in 400 ml konz. Schwefelsäure, wie in Beispiel 1 b) beschrieben, nitriert.

Das Reaktionsgemisch wurde mit 300 ml Wasser versetzt, 1 g Vanadiumpentoxid zugegeben und unter Einleiten von Luft (100 l/h) auf 145°C erhitzt. Bei dieser Temperatur wurden während 9 Stunden insgesamt 270 ml 65 gew.-%ige Salpetersäure zudosiert.

Das Reaktionsgemisch wurde auf Raumtemperatur abgekühlt, der ausgefallene Niederschlag abgesaugt, mit Wasser gewaschen und getrocknet.

Man erhielt 87 g (81% d. Theorie) 4-Methylsulfonyl-2-nitrobenzoesäure (Fp.: 203 bis 206°C).

## Patentansprüche

1. Verfahren zur Herstellung von Methylsulfonylbenzoesäuren der Formel I 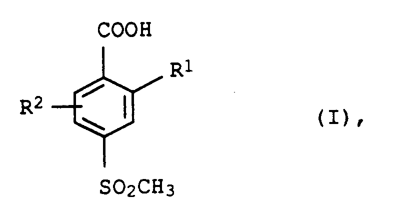 in der
R¹ Wasserstoff, Nitro, Halogen oder Hydroxysulfonyl und
R² Wasserstoff, Nitro oder Halogen bedeuten, dadurch gekennzeichnet, daß man Methylsulfonyltoluole der Formel II 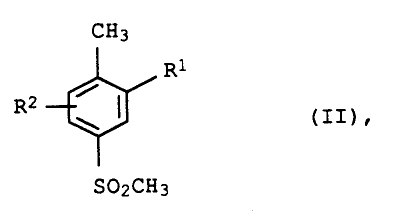
in der R¹ und R² jeweils die obengenannte Bedeutung besitzen, in Schwefelsäure in Gegenwart von Vanadium- oder Kobaltverbindungen mit Salpetersäure und Luft oxidiert.

2. Verfahren nach Anspruch 1, dadurch gekennzeichnet, daß R¹ Wasserstoff oder Nitro und R² Wasserstoff bedeuten.

3. Verfahren nach Anspruch 1, dadurch gekennzeichnet, daß man die Oxidation in Gegenwart von Vanadiumverbindungen vornimmt.

4. Verfahren nach Anspruch 1, dadurch gekennzeichnet, daß man die Oxidation bei einer Temperatur von 130 bis 170°C vornimmt.

## Claims

1. A process for preparing methylsulfonylbenzoic acids of the formula I 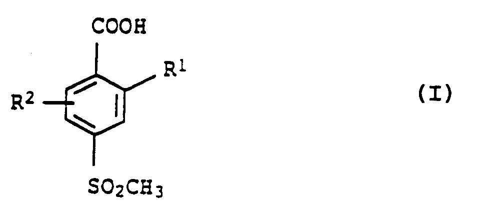 where
R¹ is hydrogen, nitro, halogen or hydroxysulfonyl, and
R² is hydrogen, nitro or halogen, which comprises oxidizing methylsulfonyltoluenes of the formula II 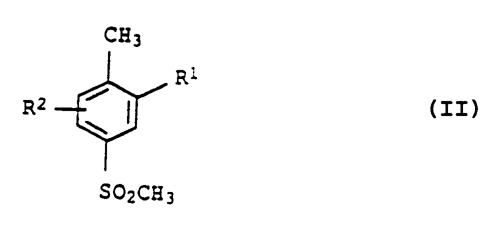
where R¹ and R² are each as defined above, in sulfuric acid with nitric acid and air in the presence of vanadium or cobalt compounds.

2. A process as claimed in claim 1, wherein R¹ is hydrogen or nitro and R² is hydrogen.

3. A process as claimed in claim 1, wherein the oxidation is carried out in the presence of vanadium compounds.

4. A process as claimed in claim 1, wherein the oxidation is carried out at from 130 to 170°C.

## Revendications

1. Procédé de préparation d'acides méthylsulfonylbenzoïques de la formule I 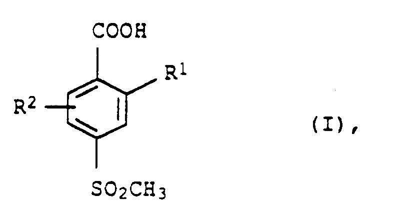 dans laquelle
R¹ représente un atome d'hydrogène, un radical nitro, un atome d'halogène, ou un radical hydroxysulfonyle et
R² représente un atome d'hydrogène, un radical nitro, ou un atome d'halogène,caractérisé en ce que l'on oxyde des méthylsulfonyltoluènes de la formule II 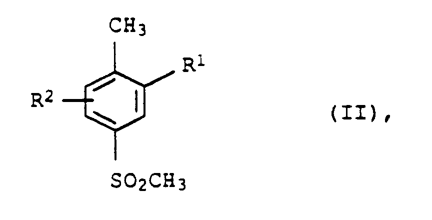
dans laquelle R¹ et R² possèdent les significations qui leur ont été attribuées ci-dessus, dans de l'acide sulfurique, en présence de composés du vanadium ou du cobalt, avec de l'acide nitrique et de l'air.

2. Procédé suivant la revendication 1, caractérisé en ce que R¹ représente un atome d'hydrogène ou le radical nitro et R² représente un atome d'hydrogène.

3. Procédé suivant la revendication 1, caractérisé en ce que l'on entreprend l'oxydation en présence de composés du vanadium.

4. Procédé suivant la revendication 1, caractérisé en ce que l'on entreprend l'oxydation à une température de 130 à 170°C.
